# EUROPEAN PATENT APPLICATION

(11) **EP 4 005 520 A1**
(43) Date of publication of application: **01.06.2022**
(21) Application number: 21211458.1
(22) Date of filing: 30.11.2021
(51) Int. Cl.: A61B 34/00, A61B 34/10, A61B 34/32, A61B 34/20

(54) **HYBRID CONTROL OF SURGICAL ROBOT FOR FAST POSITIONING ONTO PLANNED TRAJECTORIES**

(30) Priority: 30.11.2020 US 202063119414 P
(71) Applicant: Medtech SA, 34000 Montpellier (FR)
(72) Inventor: PITHON, Thomas, Montpellier (FR); SINTES, Arnaud, Montpellier (FR); GOUILLART, Romain, Montpellier (FR)
(74) Representative: Taor, Simon Edward William

(57) **Abstract**

Systems and methods may provide autonomous robotic control. For example, a method may include planning a surgical procedure including receiving identification of a key point. The method may include determining a trajectory for a robotic arm, for example based on the key point. In an example, the trajectory includes a position and orientation for the robotic arm. The method may include identifying that the robotic arm has been moved into an influence zone corresponding to the key point, and controlling the robotic arm to move, in an autonomous mode, to be aligned along the trajectory.

## Description

### CLAIM OF PRIORITY

This application claims the benefit of priority to U.S. Provisional Application No. 63/119,414 filed July 15, 2020, titled "HYBRID CONTROL OF SURGICAL ROBOT FOR FAST POSITIONING ONTO PLANNED TRAJECTORIES," which is hereby incorporated herein by reference in its entirety.

### BACKGROUND

The use of robotics in surgery is on the rise as more procedures are using robotics to positively affect surgical outcomes. Robotic surgery may be used for orthopedic or neurosurgical procedures, such as knee, shoulder, hip, spine, brain, etc., surgical procedures. While some techniques exist for using robotics to control surgical tools, these techniques may come with high costs, specialized equipment, longer surgical planning times, or longer surgical operation times.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a robotic surgery system in accordance with some embodiments.
FIG. 2 illustrates a surgical workflow including a hybrid mode in accordance with some embodiments.
FIG. 3 illustrates a cooperative mode diagram for use with a robotic surgical device in accordance with some embodiments.
FIG. 4 illustrates a diagram showing planned object identification in accordance with some embodiments.
FIG. 5 illustrates an automatic mode diagram for use with a robotic surgical device in accordance with some embodiments.
FIG. 6 illustrates a user interface for use with a robotic surgical device in accordance with some embodiments.
FIG. 7 illustrates a flowchart showing a technique for performing a robotic assisted surgical procedure in accordance with some embodiments.
FIG. 8 illustrates generally an example of a block diagram of a machine upon which any one or more of the techniques discussed herein may perform in accordance with some embodiments.

### DETAILED DESCRIPTION

Systems and methods for using robotic systems and planning software during a surgical procedure, such as spine surgeries, neurosurgeries, orthopedic and any other type of surgeries are described herein. A robotic surgical device may be used in a surgical procedure to assist a surgeon in completing the surgical procedure. The robotic surgical device may operate according to one or more modes, such as a cooperative mode, an autonomous mode, a safety mode, a diagnostic mode, an initialization mode, or the like.

Robotic movement may be autonomous, meaning the robotic surgical device moves a portion, such as a robotic arm, without direct guidance from a human. The autonomous movement may be pre-programmed or determined based on a set of parameters, such as current location of the robotic surgical device, location of other areas of interest (e.g., patient anatomy, instruments, etc.), type of procedure, trajectory, safety zones, or the like. Autonomous robotic movement may include movement initiated by a command, such as a button press, a voice command, a foot pedal, selection of a user interface component, or the like.

Robotic movement may be cooperative, such as including direct guidance from a human. The direct guidance may include surgeon control of a robotic arm, for example by the surgeon physically touching and moving the robotic arm or an instrument or device coupled to the robotic arm. The cooperative movement may include a force assist type of movement, where a light touch by the surgeon is augmented by the power control of the robotic arm to move the robotic arm in a particular direction of the light touch, which may be identified from a sensor of the robotic surgical device. In another example, the cooperative movement may include a force resist type of movement. In a force resist setup, the robotic surgical device may resist movement by a surgeon outside a designated area (e.g., a zone), outside a plane or line, or away from a designated path or trajectory. In an example, force assistance and force resistance may be used together (e.g., force assist for movement, but preventing movement outside a zone) or sequentially, for example in different zones.

A current mode of the robotic surgical device may be changed based on a voice command, foot pedal, a location of a portion of the robotic surgical device (e.g., a robotic arm end effector or device attached to the end effector) such as a particular zone, a selection on a user interface (e.g., a virtual or augmented reality user interface or a user interface on a display screen), a temporal factor (e.g., after a specified period of time has elapsed), identification that a procedure or portion of a procedure has been completed, based on a control configuration (e.g., an algorithm, a program, a set of instructions, etc.), or the like.

In an example two robotic movement modes (automatic and cooperative modes) may be automatically changed based on detection of whether a selected planned object is inside or outside an influence zone. Visual feedback (e.g., on a user interface) or auditory feedback may be provided to notify a surgeon of a change or upcoming change in modes. For example, an alert may be output to identify that a planned object is inside or outside an influence zone, or that a switch from a robot movement mode to another mode has occurred.

Robotized assisted surgical techniques may use planning data to cause a surgical instrument, for example attached to the end effector of a robotic arm, onto a trajectory defined in a surgical plan. The trajectory may include an entry and a target point relative to anatomy of a patient. When the robotic arm moves the surgical instrument in the planned trajectory, the robotic arm may move from a current or initial position and orientation to a planned position and orientation related to the trajectory defined in the surgical planning software. The current or initial position and orientation may include a home position of the robotic arm when transitioning to a first trajectory, or a planned trajectory from a specified location (e.g., a safe zone location) when transitioning to a second or subsequent trajectory. The movement may include avoidance of obstacles located between two different trajectories, such as by moving first to a safe zone. The safe zone may include a safe trajectory (e.g., to a location where linear or planar movement to a target is not blocked by an obstacle).

Some techniques may include automatically moving the robotic arm directly from a planned trajectory to another one following a specific workflow. For example, in the case of a spine surgery, the workflow may be to move along planned trajectories from the right-side patient bottom to the right-side patient top, and then to the left side patient bottom to the left side patient top. A drawback of this technique is its complex setup requirements, since as there are so many different workflows according to type of surgery, patient anatomy and pathology, surgeon preferences, and surgical room layout. This technique may not prevent from collision with surgical materials.

Some techniques may include automatically moving the robotic arm to an intermediary position between two planned trajectories. This intermediary position may be defined in function of the type of surgery and the operating room layout. A drawback of this technique is that it slows down the surgery process. Speed of the robotic arm movement must be slow for safety reasons as it is close to patient anatomy or surgical instrument.

Some techniques may include using a force sensor embedded on an end effector of the robotic arm to cooperatively move the robotic arm until it reaches a specified trajectory. Other sensors may be used in other examples, such as a capacitive sensor or a proximity sensor. A planning user interface may provide (e.g., in real time) the movement of the robotic arm (for example by following a visual representation of it on the user interface) relative to the anatomy and planned trajectories. However, a drawback of this technique is the complicated preparation required to accurately and quickly reach the planned trajectory.

In a technique that solves these technical problems, the robotic arm may be caused to move onto a planned trajectory more quickly and accurately to accelerate the surgical workflow without losing accuracy, along with better usability to be more adapted to the surgical context. The technique described herein may be applicable to any type of surgery, patient anatomy and pathology, surgeon preferences, or surgical room layout. In an example, the robotic arm may be moved cooperatively from a first trajectory (after completing a surgical procedure step) to a second trajectory. At each trajectory, the robotic arm may autonomously move along the trajectory. For example, the robotic arm may be used to perform a spine surgery procedure, with each step having a different trajectory, corresponding to a surgical step at different locations.

In some examples, such as for spine or brain procedures, the robotic arm may autonomously maintain a starting point of the trajectory. For example, in a brain procedure, this starting point is fixed. In a spine procedure, this starting point is maintained relative to patient movements, and thus the robotic arm moves in response to the patient movements. In brain or spine procedures, the surgeon instrument may move via the surgeon's hand moving the instrument along the trajectory. Using the robotic end effector, the surgeon may know the instrument is being maintained along the trajectory and reach the trajectory endpoint without going further. In other examples, such as for knee or a spine procedure, the robotic arm may move autonomously along the trajectory.

The systems and methods described herein that include embodiments of this technical solution use a human and robot cooperative movement, with the aid of data provided by an embedded force sensor, to quickly move the robotic arm near planned trajectories. Automatic robotic movement may be activated automatically to reach accurately planned trajectories, for example after the cooperative movement.

The systems and methods described herein combine two robotic control modes and may use manageable robotic virtual influent zones (e.g., a virtual 3D representation of a geometric figure that virtually surrounds the axis of the robot arm trajectory representation on the user interface or the axis of the planned trajectory) or real time user interface feedback. A user-controlled (e.g., via button, pedal, user interface component, etc.) switch between automatic robot movements and cooperative human and robot movement may be used to cause the robotic arm to reach planned trajectories. To control this switch, the surgeon may interact with a user interface component. In an example, a planned trajectory (which may be planned pre-operatively or planned or modified intra-operatively) in a highlighted manner to indicate this trajectory is inside the "robot influence zone." The surgeon may then stop moving the robotic arm, for example during a specified time, and the robotic arm may move automatically to planned coordinates along a planned trajectory. The autonomous movement may be conveyed in a message to the surgeon (e.g., on a user interface or via audio), and may optionally request a confirmation from the surgeon to commence the autonomous movement.

The combination of cooperative and autonomous robotic movement reduces the time needed for autonomous movement and increases cooperative movement time while led by the surgeon. These systems and methods further are adapted to any surgery type, patient anatomy and pathology, surgeon preferences, or surgical room layout. A robotic arm may move quicker in cooperative mode than in automatic mode (e.g., for safety reasons), which allows the combination to improve surgery time and fluidity. The extended use of the cooperative mode reduces risks of collisions between the robot arm and surgical items (e.g., by allowing the surgeon to navigate around obstacles), and allows the surgeon to better control the robotic arm movement when initiating movement onto planned trajectories.

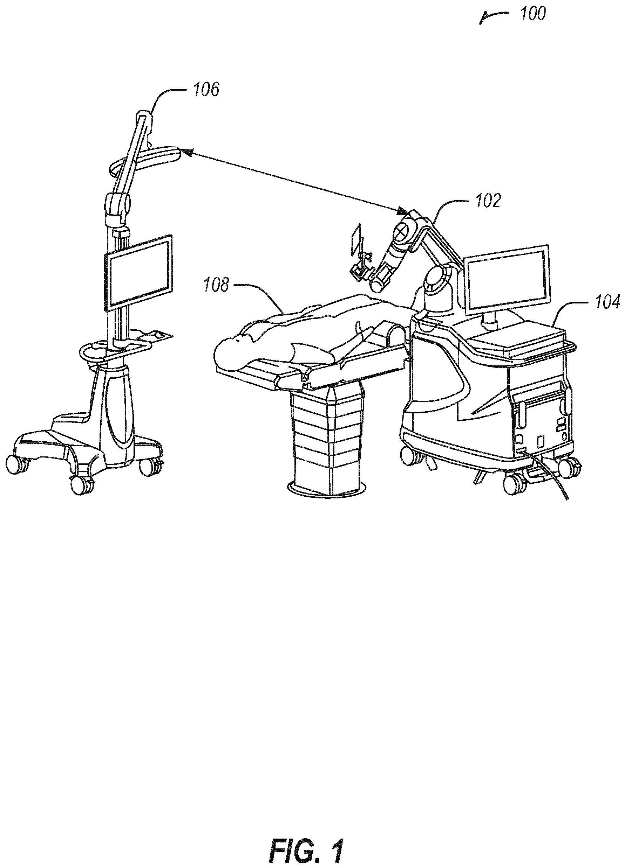

FIG. 1 illustrates a robotic surgery system 100 in accordance with some embodiments. The system 100 includes a robotic surgical device 102, which may include a computing device 104 (e.g., a processor and memory for performing instructions). The robotic surgical device 102 may be used to perform a portion of a surgical procedure on a patient 108 (e.g., a partial or total knee arthroplasty, a hip arthroplasty, a shoulder arthroplasty, a spine procedure, a brain surgery, etc.). The system 100 may include a display device 106, which may be used to issue an alert, display information about a procedure or trajectory, or receive surgeon input, such as a selection or confirmation.

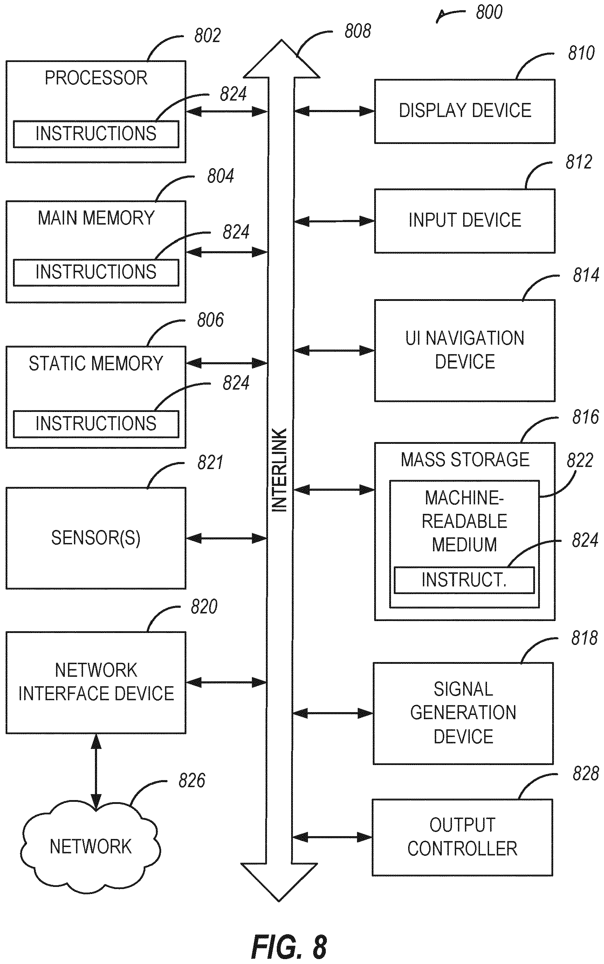

The robotic surgical device 102 may include a robotic arm, including an end effector. A device, such as an implant, cut guide, surgical instrument, or the like, may be affixed or attached to the end effector. The robotic surgery system 100 may include processing circuitry to implement a control system. The processing circuitry may include the computing device 104, circuitry embedded within or attached to the robotic arm or other portion of the robotic surgical device 102, software (e.g., configured to run on the computing device 104), or the like. The computing device 104 may include a computer, peripheral devices, power supply, a motor, communication circuitry, or the like (e.g., as described below with respect to FIG. 8).

The robotic surgical device 102 may include a tracking system. The tracking system may optionally include a camera or a sensor (e.g., an infrared sensor, an optical sensor, etc.). The tracking system may use the camera or the sensor to track the robotic arm, the end effector, a device affixed to the end effector, a target object, or the like. In an example, the tracking system may be used to determine a position or an orientation of the robotic arm or the end effector. The position or the orientation may be determined relative to a coordinate system or relative to a target object. The identified position or orientation may be used to display a representation on a display screen or augmented or mixed reality view of the robotic arm or end effector. The representation may be displayed with respect to a key point, planned object, trajectory, or patient anatomy, as described herein.

The optional tracking system allows the robotic arm or surgeon instrument position to be tracked or patient movements to be tracked. Tracking targets (e.g., infrared reflectors) may be set on a base of the robotic arm, a surgery instrument (screwdriver for example), or a portion of the patient (e.g., pinned onto the illiac crest or via a spinous clamp for a spine procedure for example, or pinned on the tibia and femur for a knee procedure). A mathematical registration may be performed to express position or orientation of surgery instruments in the patient target referential to the image referential. The position or orientation may be displayed on a screen, such as with surgery instruments co-localized within the patient imagery (e.g., for a spine procedure). In an example, the robotic arm position or orientation is tracked by the robotic system. In this example, the robotic arm position or orientation may be expressed in the camera referential by using an infrared target on the robotic base. A mathematical transformation may be used to transform a position or orientation in a robotic coordinate space to a camera coordinate space.

The robotic surgical device 102 may be operated in one or more modes. In an example, a mode may be switched on the fly during surgery (e.g., automatically based on a location of the end effector or the robotic arm, based on a surgeon input, such as on a user interface, a foot pedal, a button, a voice command, or the like). For example, the surgeon may switch the robotic surgical device 102 between operation in a cooperative mode and an automatic mode, such as to control the end effector onto a planned trajectory. The cooperative mode is a control mode that permits the surgeon to manually move the robotic arm. Using a force sensor, the robotic surgical device 102 may recognize the forces applied by the surgeon onto the robot arm or end effector, and the robotic arm may move along this force (e.g., using a motor to control movement of the robotic surgical device 102). The automatic mode allows the robotic arm to move automatically, for example from a starting position and orientation to an ending position and orientation.

The surgeon may manage the use of the robotic control modes with the aid of a user interface. In an example, the user interface may show, for example in real time, the position and orientation of the robot arm end effector, the planned trajectories defined by an entry and a target point relative to the patient anatomy, the elected planned trajectory, the robot arm control mode, a current surgical operation, or the like.

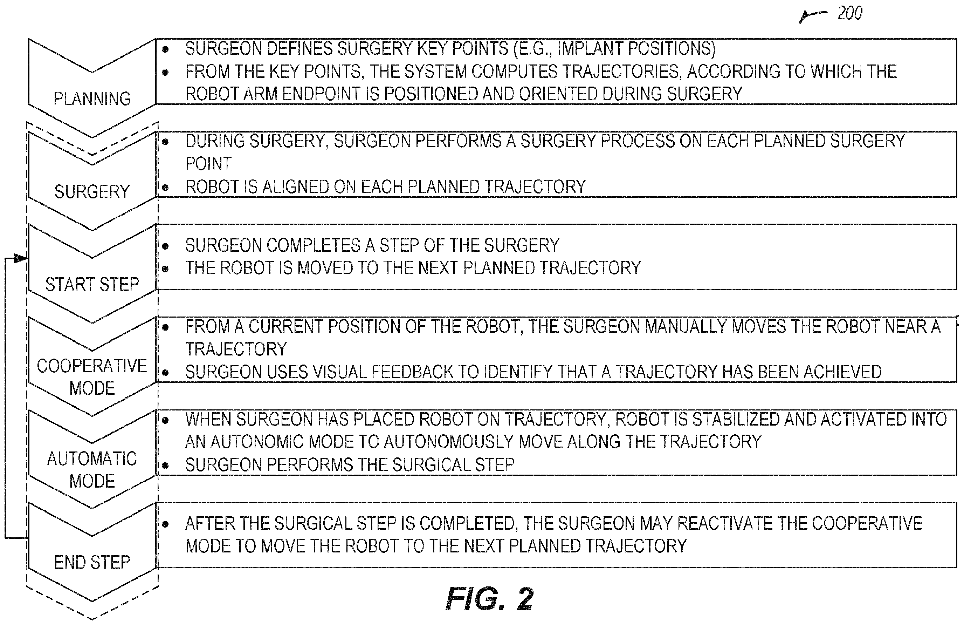

FIG. 2 illustrates a surgical workflow 200 including a controlling operation of a robotic device in a hybrid mode in accordance with some embodiments. The hybrid mode includes an autonomous mode and a cooperative mode, in an example. The operations in the surgical workflow 200 depicted in FIG. 2 may be performed in different orders, and one or more operations may be omitted, in some examples.

The surgical workflow 200 includes a preoperative or intraoperative portion, including a planning operation, which may be part of a larger surgical planning operation. The planning operation of the surgical workflow 200 includes receiving surgeon-defined key points, which may be input manually on a user interface or collected via an optical pointer or other input device. The key points may include an implant position (either a present implant position or a planned future position of an implant), an anatomy point, a reference point, or the like. Based on the received key points, a trajectory may be determined for a robotic arm to move an end effector to or from during a surgical procedure. The trajectory may include a position and orientation of an initial or final position of the end effector. A plurality of trajectories may be determined for a surgical procedure. Intraoperative planning may be a planning after patient opening for example or a planning when the patient is on the surgery table and under anesthesia.

The surgical workflow 200 includes an intra-operative portion, which includes a plurality of operations. These operations include aligning the robotic arm (e.g., aligning the end effector) on a plurality of trajectories during the surgical procedure. This process includes starting a surgical step by moving (in a cooperative mode) the robotic arm onto a next trajectory. In the initial case, the surgeon may start the surgical procedure by moving the robotic arm from a starting position to a first trajectory. In later cases, the surgeon may move the robotic arm, after completion of a previous surgical operation, to a next trajectory.

While aligning the robotic arm (in a cooperative mode) with a next trajectory, a user interface may display a representation of the robotic arm to indicate where the robotic arm is located relative to planned key points. In another example, no representation of the robotic arm may be displayed. In either case, an indication may be provided when the robotic arm is near a key point, at a key point, aligned along a trajectory, or within a particular key point zone. The indication may include a visual indication on a user interface, such as a flashing light or written indication, an audible indication (e.g., audio tone), haptic feedback, or the like. In an example, the indication may be provided directly by the robotic arm, which may lock in place (e.g., by resisting movement) or lock along a plane or line. In an example, the user interface may require selection of a key point.

The user interface may display a confirmation request, which when selected may move the robotic device to automatic mode. In another example, the robotic arm may switch to an autonomous mode automatically when in place along a trajectory (e.g., when the surgeon selects the trajectory before moving the robotic arm). The surgeon may optionally stabilize the robotic arm at the trajectory (e.g., at the key point). The surgical workflow 200 includes an operation to autonomously move the robotic arm (e.g., via processing circuitry implementing a control system) in response to receiving a confirmation or in response to the robotic arm switching to an autonomous mode. When the portion of the surgical procedure corresponding to the current trajectory is completed, the surgeon may reactivate the cooperative mode to move the robotic arm to the next planned trajectory.

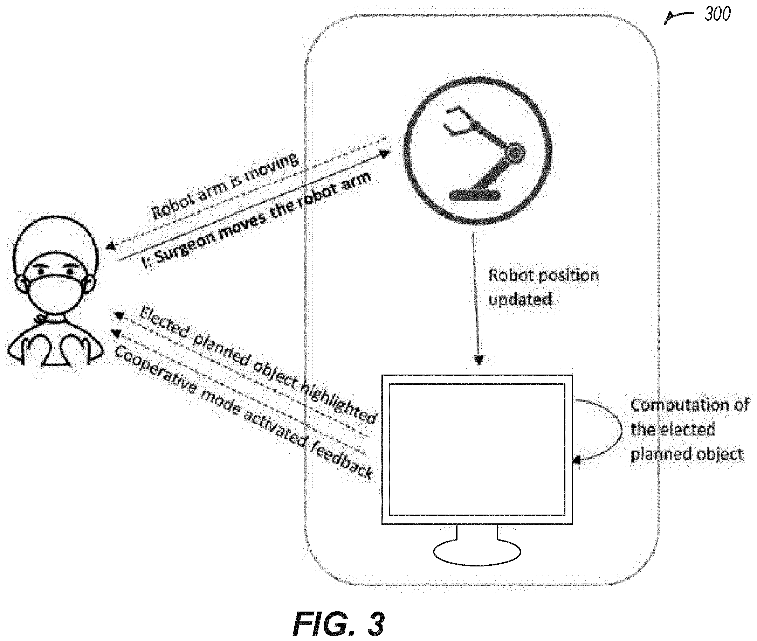

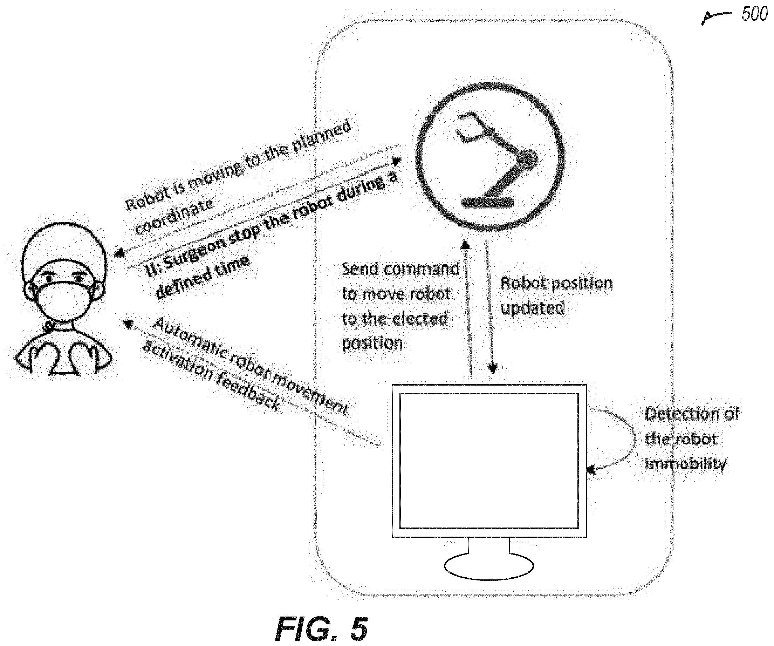

FIG. 3 illustrates a cooperative mode diagram 300 for use with a robotic surgical device in accordance with some embodiments. The cooperative mode diagram 300 illustrates actions that may be taken when the robotic arm is in a cooperative mode. In an example, while in the cooperative mode, the robotic arm does not move without surgeon force applied to the robotic arm. To align the robot arm onto a planned trajectory, the surgeon may follow a two steps process. The cooperative mode diagram 300 shows the first step where the surgeon uses the cooperative mode to move the robotic arm near the chosen planned trajectory. The robotic arm sends information about its current position periodically (e.g., every second, every millisecond, in real-time, or the like). A user interface may be updated (e.g., on a real time basis) to show a current position or orientation of the robotic arm. The sure interface may display a currently selected planned object, such as a key point, a trajectory, or the like. Using the visual feedback of the user interface and moving the robotic arm in the cooperative mode, the surgeon may place the robotic arm at a key point or aligned along a trajectory. The second step is illustrated in FIG. 5, below, when the robotic arm is placed in an autonomous mode.

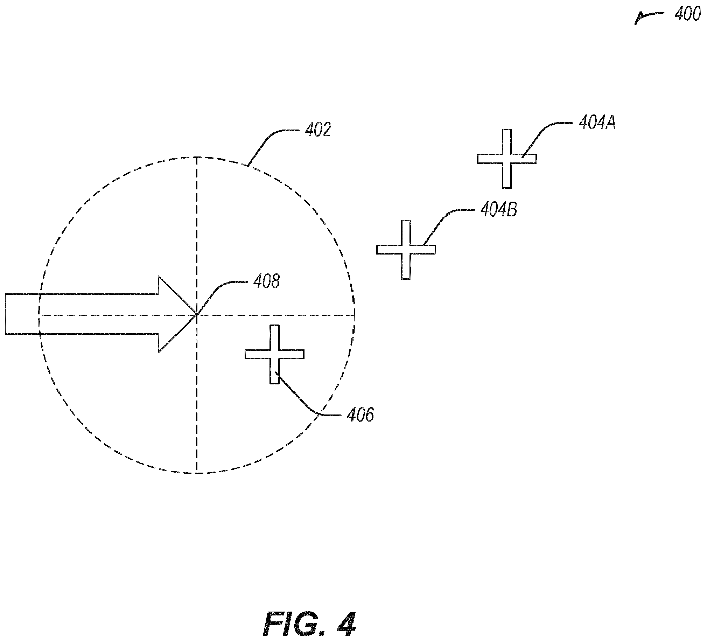

FIG. 4 illustrates a user interface diagram 400 showing planned object identification in accordance with some embodiments. In a planning operation, a plurality of key points may be selected, which may correspond to trajectories a robotic arm will move along during a surgical procedure. The planned trajectories may be represented by objects on a user interface. The planned trajectories may be ordered, semi-ordered (e.g., a specific trajectory may be contingent on completion of another specific trajectory), or un-ordered.

The user interface diagram 400 illustrates both an elected planned object 406 and non-elected planned objects 404A and 404B, but in some examples non-elected planned objects 404A or 404B may be hidden from view. The elected planned object 406 may be displayed in a manner that is visually distinct from non-elected planned objects 404A and 404B. The user interface diagram 400 shows a robotic arm trajectory axis 408 (e.g., line), which may be a 2D or 3D axis.

The robotic arm may be moved in a cooperative mode (e.g., as described above with respect to FIG. 3), until the robotic arm is moved within a range of the elected planned object 406. The user interface may change a visual representation of the elected planned object 406 when the robotic arm is in range. The user interface may provide an indication, for example instructing the surgeon to stop moving the robotic arm. The robotic arm may align itself with the planned trajectory automatically once in range of the elected planned object 406 (e.g., autonomously move into alignment with the trajectory).

The range surrounding the elected planned object 406 may be called an influence zone 402. The influence zone 402 may include a specified area (e.g., a circle, sphere, square, cube, cone, or other geometric shape around the planned object), which may be personalized to the present surgical procedure, specific to the planned object, or general. The influence zone 402 may be sized based on how close the planned objects or trajectories corresponding to the planned objects are to each other.

The computation of the elected planned object 406 may be performed by defining an influence zone 402 around the robot arm trajectory. Each time a new robot arm position and orientation is received, the planned objects may be checked as to whether they are inside the influence zone 402. In another example, only the elected planned object 406 may be checked.

FIG. 4 describes how the elected planned object 406 is computed. The influence zone 402 may be located either on the robot arm trajectory or on each planned trajectory. Geometrical shape and size of the influence zone 402 may be created, modified, or adjusted during pre- or intra-operative planning, such as depending on a type of surgery. The planned objects (e.g., 404A, 404B, or 406) and influence zone 402 may be selected to minimize a number of simultaneous planned objects in the influence zone 402. In an example, the influence zone 402 includes only one planned object or trajectory. In another example, the influence zone 402 may include two or more objects, but may be limited to trajectories that are parallel or diverging. In an example, the system may prompt the surgeon to select a planned object or trajectory, in order to autonomously send the robotic arm onto the planned trajectory.

FIG. 5 illustrates an automatic mode diagram 500 for use with a robotic surgical device in accordance with some embodiments. The automatic mode diagram 500 represents a configuration after movement of the robotic arm in a cooperative mode (e.g., corresponding to the actions described above with respect to FIG. 3). The robotic arm may be switched from the cooperative mode to an automatic mode by surgeon selection (e.g., via a voice command, foot pedal, button, user interface interaction, etc.) or automatically when the robotic arm is identified as being within an influence zone (e.g., as described above with respect to FIG. 4).

After the surgeon has selected the desired planned object and moved the robotic arm into the influence zone of the planned object while the robotic arm is in the cooperative mode, the surgeon may stop moving the robotic arm. In an example, when the robotic arm does not move for a specified period of time (e.g., 1 to 10 seconds), the autonomous mode may be initiated or an indication may be provided to the surgeon. The indication may ask the surgeon to confirm entry into the autonomous mode. In another example, the indication may alert the surgeon that entry into the autonomous mode will occur (e.g., after expiration of a second period of time). Either period of time may be determined based on a surgery type or a surgeon preference.

After the surgeon confirms or the automated process completes, the robotic arm is transitioned to the autonomous mode. The robotic arm autonomously changes position and orientation automatically to reach the selected trajectory. The surgeon may then continue the surgical process. When the surgical operation using the selected trajectory is completed, the surgeon may reactivate the cooperative mode (e.g., by moving the robotic arm, by selecting an indication on the user interface, with a voice command, a foot pedal, or the like). The surgeon may cooperatively move the robotic arm to the next planned object and repeat the process.

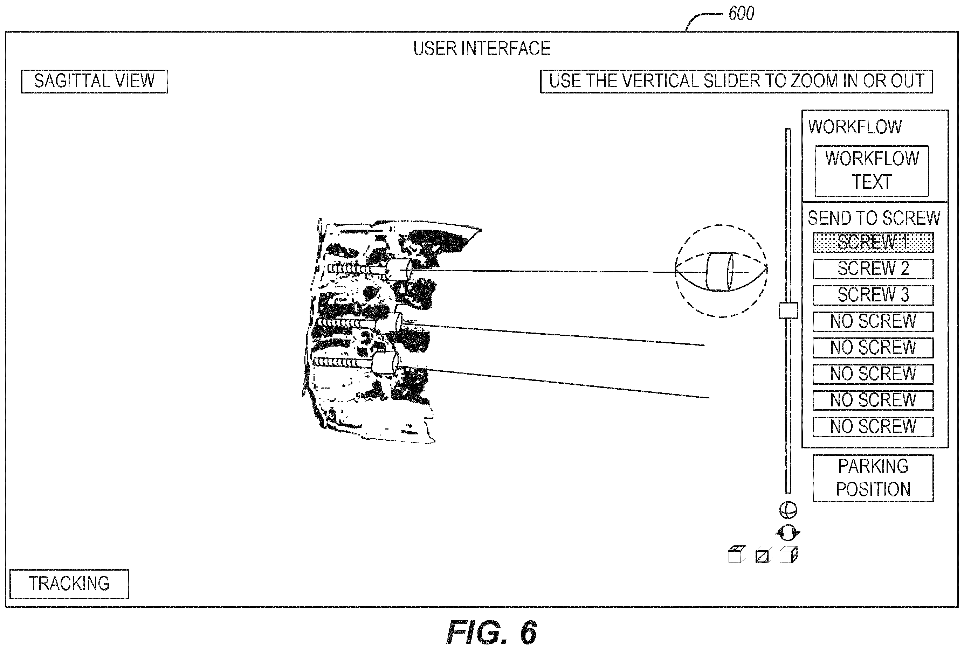

FIG. 6 illustrates a user interface 600 for use with a robotic surgical device in accordance with some embodiments. The user interface 600 may include illustrated trajectories, influence zones, key point objects, patient anatomy, representations of existing or planned implants, selectable indications, or the like.

The user interface 600 may be used to switch a robotic arm between cooperative and autonomous modes. In an example, switching from autonomous mode to cooperative mode may include removing a lock on movement of the robotic arm. The removal may occur after a surgical step is completed.

Trajectories or objects (e.g., a key point or influence zone) may be displayed on the user interface 600. In an example, only an active trajectory or object is displayed. In another example, all trajectories and objects for a surgical procedure are displayed, with an optional distinguishing visual feature for an active trajectory or object (e.g., a different color, highlight, transparency level, flashing, etc.). In some examples, trajectories or objects may be removed from the user interface 600 when they are completed (e.g., when the corresponding surgical operation is completed).

A surgeon may select a trajectory or object on the user interface 600 to activate that trajectory or object. In another example, trajectories or objects may progress sequentially according to a surgical plan. In this example, the surgical procedure may be completed without touching the user interface 600. The surgeon may change properties or aspects of a trajectory or object. For example, a location of an object may be moved, an influence zone may change size, a trajectory may be changed, or the like.

The user interface 600 may include a 3D view. The example user interface 600 shown in FIG. 6 includes a reconstructed CT scan with 3 planned pedicle screws, with three trajectories represented as 3D lines. During the surgery, the robotic arm endpoint is aligned (e.g., by the surgeon in a cooperative mode) on a starting point of each trajectories. In the example user interface 600, the starting point corresponds to the beginning of each of the lines. The cylinder depicted on the user interface 600 represents the robotic arm endpoint position and orientation, which is within an influence zone. The two circles or sphere represents the influence zone. In this example user interface 600, as the influence zone intersects the top most trajectory starting point, the selected trajectory may be highlighted or change color. An indication (e.g., on the right side of the user interface 600) may be highlighted or change color to indicate which object or surgical operation corresponds to the trajectory and influence zone currently occupied by the robotic arm or a selected trajectory or influence zone.

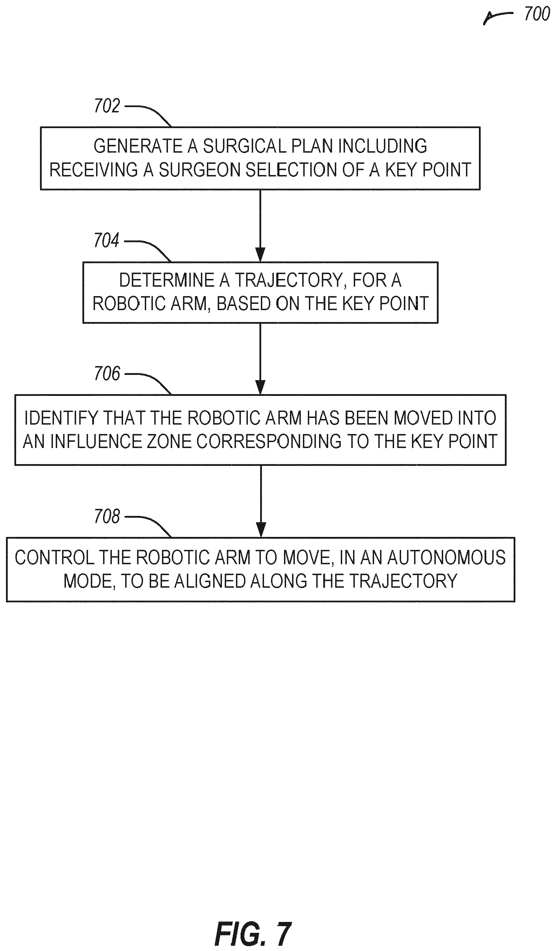

FIG. 7 illustrates a flowchart showing a technique 700 for performing a robotic assisted surgical procedure in accordance with some embodiments. The technique 700 may be implemented using a robotic surgical system, such as with a processor. The technique 700 may be performed by the processor by executing instructions stored in memory.

The technique 700 includes an operation 702 to generate a surgical plan including receiving a surgeon selection of a key point. The technique 700 includes an operation 704 to determine a trajectory, for a robotic arm, based on the key point. The trajectory may include a position, an orientation, a coordinate, an angle, a relative location, or the like, for the robotic arm. The trajectory may be planned into the surgical plan.

The technique 700 includes an operation 706 to identify that the robotic arm has been moved into an influence zone corresponding to the key point. Operation 706 may occur when the robotic arm is in a cooperative mode, such as providing force assistance, force resistance, or the like. The influence zone may include a volume, such as a regular volume (e.g., a sphere, a cube, etc.) or an irregular volume (e.g., a surgeon defined volume around the key point, an automatically generated volume around the key point based on safety zone areas, or the like). The influence zone may correspond to a step of a surgical procedure corresponding to the trajectory.

The technique 700 includes an operation 708 to control the robotic arm to move, in an autonomous mode, to be aligned along the trajectory. Operation 708 may include controlling the robotic arm to operate below a maximum speed in each of the cooperative and autonomous modes, the maximum speed being higher in the cooperative mode than the autonomous mode. Technique 700 may include displaying a user interface on a display device, the user interface including a visual representation of the key point or the trajectory.

In an example, the technique 700 includes receiving a surgeon selection to proceed with the autonomous movement along the trajectory before controlling the robotic arm to be aligned along the trajectory. Receiving the surgeon selection may include receiving selection on the user interface of the key point from a plurality of planned key points corresponding to a plurality of planned trajectories. In this example, after selection of the key point, the technique 700 may include displaying the key point on the user interface in a visually distinct manner from remaining key points of the plurality of key points.

The technique 700 may include executing actions after completion of a step of a surgical procedure corresponding to the trajectory. The actions may include, for example, removing the key point from display on the user interface, reactivating the cooperative mode for the robotic arm, or activating a new key point on the user interface. The technique 700 may include receiving tracking information of movement of the robotic arm. In an example, a position or orientation of the robotic arm corresponding to the tracking information may be displayed on the user interface. The technique 700 may include receiving an intraoperative change to the trajectory before the autonomous movement, the change causing the robotic arm to autonomously move the robotic arm along the new trajectory.

FIG. 8 illustrates generally an example of a block diagram of a machine 800 upon which any one or more of the techniques (e.g., methodologies) discussed herein may perform in accordance with some embodiments. In alternative embodiments, the machine 800 may operate as a standalone device or may be connected (e.g., networked) to other machines. In a networked deployment, the machine 800 may operate in the capacity of a server machine, a client machine, or both in server-client network environments. The machine 800 may be a personal computer (PC), a tablet PC, a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate on, logic or a number of components, modules, or like mechanisms. Such mechanisms are tangible entities (e.g., hardware) capable of performing specified operations when operating. In an example, the hardware may be specifically configured to carry out a specific operation (e.g., hardwired). In an example, the hardware may include configurable execution units (e.g., transistors, circuits, etc.) and a computer readable medium containing instructions, where the instructions configure the execution units to carry out a specific operation when in operation. The configuring may occur under the direction of the executions units or a loading mechanism. Accordingly, the execution units are communicatively coupled to the computer readable medium when the device is operating. For example, under operation, the execution units may be configured by a first set of instructions to implement a first set of features at one point in time and reconfigured by a second set of instructions to implement a second set of features.

Machine (e.g., computer system) 800 may include a hardware processor 802 (e.g., a central processing unit (CPU), a graphics processing unit (GPU), a hardware processor core, or any combination thereof), a main memory 804 and a static memory 806, some or all of which may communicate with each other via an interlink (e.g., bus) 808. The machine 800 may further include a display unit 810, an alphanumeric input device 812 (e.g., a keyboard), and a user interface (UI) navigation device 814 (e.g., a mouse). In an example, the display unit 810, alphanumeric input device 812 and UI navigation device 814 may be a touch screen display. The display unit 810 may include goggles, glasses, or other AR or VR display components. For example, the display unit may be worn on a head of a user and may provide a heads-up-display to the user. The alphanumeric input device 812 may include a virtual keyboard (e.g., a keyboard displayed virtually in a VR or AR setting.

The machine 800 may additionally include a storage device (e.g., drive unit) 816, a signal generation device 818 (e.g., a speaker), a network interface device 820, and one or more sensors 821, such as a global positioning system (GPS) sensor, compass, accelerometer, or other sensor. The machine 800 may include an output controller 828, such as a serial (e.g., universal serial bus (USB), parallel, or other wired or wireless (e.g., infrared (IR), near field communication (NFC), etc.) connection to communicate or control one or more peripheral devices.

The storage device 816 may include a machine readable medium 822 that is non-transitory on which is stored one or more sets of data structures or instructions 824 (e.g., software) embodying or utilized by any one or more of the techniques or functions described herein. The instructions 824 may also reside, completely or at least partially, within the main memory 804, within static memory 806, or within the hardware processor 802 during execution thereof by the machine 800. In an example, one or any combination of the hardware processor 802, the main memory 804, the static memory 806, or the storage device 816 may constitute machine readable media.

While the machine readable medium 822 is illustrated as a single medium, the term "machine readable medium" may include a single medium or multiple media (e.g., a centralized or distributed database, or associated caches and servers) configured to store the one or more instructions 824.

The term "machine readable medium" may include any medium that is capable of storing, encoding, or carrying instructions for execution by the machine 800 and that cause the machine 800 to perform any one or more of the techniques of the present disclosure, or that is capable of storing, encoding or carrying data structures used by or associated with such instructions. Non-limiting machine readable medium examples may include solid-state memories, and optical and magnetic media. Specific examples of machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 824 may further be transmitted or received over a communications network 826 using a transmission medium via the network interface device 820 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, as the personal area network family of standards known as Bluetooth^{®} that are promulgated by the Bluetooth Special Interest Group, peer-to-peer (P2P) networks, among others. In an example, the network interface device 820 may include one or more physical jacks (e.g., Ethernet, coaxial, or phone jacks) or one or more antennas to connect to the communications network 826. In an example, the network interface device 820 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 800, and includes digital or analog communications signals or other intangible medium to facilitate communication of such software.

Example 1 is a system for autonomous robotic control comprising: a robotic arm; processing circuitry configured to: implement a surgical planning device configured to receive a surgeon selection of a key point via a user interface; determine a trajectory, for the robotic arm, based on the key point, the trajectory including a position and orientation for the robotic arm; identify that the robotic arm has been moved, while in a cooperative mode, into an influence zone corresponding to the key point; and cause a control system to control the robotic arm to autonomously move, in an autonomous mode, to be aligned along the trajectory.

In Example 2, the subject matter of Example 1 includes, a display device configured to display the user interface, the user interface further including a visual representation of the trajectory.

In Example 3, the subject matter of Examples 1-2 includes, wherein the processing circuitry is further configured to receive a surgeon selection to proceed with the autonomous movement along the trajectory before causing the control system to control the robotic arm.

In Example 4, the subject matter of Example 3 includes, wherein to receive the surgeon selection includes to receive selection on the user interface of the key point from a plurality of planned key points corresponding to a plurality of planned trajectories.

In Example 5, the subject matter of Example 4 includes, wherein, after selection of the key point is received, the key point is displayed on the user interface in a visually distinct manner from remaining key points of the plurality of key points.

In Example 6, the subject matter of Examples 1-5 includes, wherein after completion of a step of a surgical procedure corresponding to the trajectory, the processing circuitry is further configured to remove the key point from display on the user interface.

In Example 7, the subject matter of Examples 1-6 includes, wherein after completion of a step of a surgical procedure corresponding to the trajectory, the processing circuitry is further configured to reactivate the cooperative mode for the robotic arm.

In Example 8, the subject matter of Examples 1-7 includes, wherein the influence zone is a volume surrounding the key point corresponding to a step of a surgical procedure corresponding to the trajectory.

In Example 9, the subject matter of Examples 1-8 includes, wherein the processing circuitry is further configured to receive tracking information of movement of the robotic arm, and wherein the user interface is configured to display a position and orientation of the robotic arm corresponding to the tracking information.

In Example 10, the subject matter of Examples 1-9 includes, wherein the processing circuitry is further configured to receive an intraoperative change to the trajectory before the autonomous movement, the change causing the control system to control the robotic arm to autonomously move the robotic arm along the new trajectory.

In Example 11, the subject matter of Examples 1-10 includes, wherein the robotic arm is configured to operate below a maximum speed in each of the cooperative and autonomous modes, the maximum speed being higher in the cooperative mode than the autonomous mode.

Example 12 is a method for autonomous robotic control comprising: generating a surgical plan including receiving a surgeon selection of a key point; determine a trajectory, for a robotic arm, based on the key point, the trajectory including a position and orientation for the robotic arm; identifying that the robotic arm has been moved, while in a cooperative mode, into an influence zone corresponding to the key point; control the robotic arm to move, in an autonomous mode, to be aligned along the trajectory.

In Example 13, the subject matter of Example 12 includes, displaying the user interface on a display device, the user interface further including a visual representation of the trajectory.

In Example 14, the subject matter of Examples 12-13 includes, receiving a surgeon selection to proceed with the autonomous movement along the trajectory before controlling the robotic arm to be aligned along the trajectory.

In Example 15, the subject matter of Example 14 includes, wherein receiving the surgeon selection includes receiving selection on the user interface of the key point from a plurality of planned key points corresponding to a plurality of planned trajectories.

In Example 16, the subject matter of Example 15 includes, after selection of the key point is received, displaying the key point on the user interface in a visually distinct manner from remaining key points of the plurality of key points.

In Example 17, the subject matter of Examples 12-16 includes, after completion of a step of a surgical procedure corresponding to the trajectory, removing the key point from display on the user interface.

In Example 18, the subject matter of Examples 12-17 includes, after completion of a step of a surgical procedure corresponding to the trajectory, reactivating the cooperative mode for the robotic arm.

In Example 19, the subject matter of Examples 12-18 includes, wherein the influence zone is a volume surrounding the key point corresponding to a step of a surgical procedure corresponding to the trajectory.

In Example 20, the subject matter of Examples 12-19 includes, receiving tracking information of movement of the robotic arm, and displaying, on the user interface, a position and orientation of the robotic arm corresponding to the tracking information.

In Example 21, the subject matter of Examples 12-20 includes, receiving an intraoperative change to the trajectory before the autonomous movement, the change causing the robotic arm to autonomously move the robotic arm along the new trajectory.

In Example 22, the subject matter of Examples 12-21 includes, wherein controlling the robotic arm includes controlling the robotic arm to operate below a maximum speed in each of the cooperative and autonomous modes, the maximum speed being higher in the cooperative mode than the autonomous mode.

Example 23 is at least one machine-readable medium including instructions for operation of a robotic arm during a surgical procedure, which executed by a processor, cause the processor to perform operations to: implement a surgical planning device configured to receive a surgeon selection of a key point via a user interface; determine a trajectory, for the robotic arm, based on the key point, the trajectory including a position and orientation for the robotic arm; identify that the robotic arm has been moved, while in a cooperative mode, into an influence zone corresponding to the key point; and cause a control system to control the robotic arm to autonomously move, in an autonomous mode, to be aligned along the trajectory.

In Example 24, the subject matter of Example 23 includes, wherein the instructions further cause the processor to output the user interface for display on a display deice, the user interface further including a visual representation of the trajectory.

In Example 25, the subject matter of Examples 23-24 includes, wherein the instructions further cause the processor to receive a surgeon selection to proceed with the autonomous movement along the trajectory before causing the control system to control the robotic arm.

In Example 26, the subject matter of Example 25 includes, wherein to receive the surgeon selection includes to receive selection on the user interface of the key point from a plurality of planned key points corresponding to a plurality of planned trajectories.

In Example 27, the subject matter of Example 26 includes, wherein, after selection of the key point is received, the key point is displayed on the user interface in a visually distinct manner from remaining key points of the plurality of key points.

In Example 28, the subject matter of Examples 23-27 includes, wherein after completion of a step of a surgical procedure corresponding to the trajectory, the instructions further cause the processor to remove the key point from display on the user interface.

In Example 29, the subject matter of Examples 23-28 includes, wherein after completion of a step of a surgical procedure corresponding to the trajectory, the instructions further cause the processor to reactivate the cooperative mode for the robotic arm.

In Example 30, the subject matter of Examples 23-29 includes, wherein the influence zone is a volume surrounding the key point corresponding to a step of a surgical procedure corresponding to the trajectory.

In Example 31, the subject matter of Examples 23-30 includes, wherein the instructions further cause the processor to receive tracking information of movement of the robotic arm, and wherein the user interface is configured to display a position and orientation of the robotic arm corresponding to the tracking information.

In Example 32, the subject matter of Examples 23-31 includes, wherein the instructions further cause the processor to receive an intraoperative change to the trajectory before the autonomous movement, the change causing the control system to control the robotic arm to autonomously move the robotic arm along the new trajectory.

In Example 33, the subject matter of Examples 23-32 includes, wherein the robotic arm is controlled to operate below a maximum speed in each of the cooperative and autonomous modes, the maximum speed being higher in the cooperative mode than the autonomous mode.

In Example 34, the subject matter of Examples 1-33 includes, wherein the surgical planning device is implemented in a robotic device including the robotic arm or a communicatively connected computer.

Example 35 is at least one machine-readable medium including instructions that, when executed by processing circuitry, cause the processing circuitry to perform operations to implement of any of Examples 1-34.

Example 36 is an apparatus comprising means to implement of any of Examples 1-34.

Example 37 is a system to implement of any of Examples 1-34.

Example 38 is a method to implement of any of Examples 1-34.

Method examples described herein may be machine or computer-implemented at least in part. Some examples may include a computer-readable medium or machine-readable medium encoded with instructions operable to configure an electronic device to perform methods as described above. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A system for autonomous robotic control comprising:
a robotic arm;
processing circuitry configured to:
implement a surgical planning device configured to receive a surgeon selection of a key point via a user interface;
determine a trajectory, for the robotic arm, based on the key point, the trajectory including a position and orientation for the robotic arm;
identify that the robotic arm has been moved, while in a cooperative mode, into an influence zone corresponding to the key point; and
cause a control system to control the robotic arm to autonomously move, in an autonomous mode, to be aligned along the trajectory.

2. The system of claim 1, further comprising a display device configured to display the user interface, the user interface further including a visual representation of the trajectory.

3. The system of claim 1, wherein the processing circuitry is further configured to receive a surgeon selection to proceed with the autonomous movement along the trajectory before causing the control system to control the robotic arm.

4. The system of claim 3, wherein to receive the surgeon selection includes to receive selection on the user interface of the key point from a plurality of planned key points corresponding to a plurality of planned trajectories.

5. The system of claim 4, wherein, after selection of the key point is received, the key point is displayed on the user interface in a visually distinct manner from remaining key points of the plurality of key points.

6. The system of claim 1, wherein after completion of a step of a surgical procedure corresponding to the trajectory, the processing circuitry is further configured to remove the key point from display on the user interface.

7. The system of claim 1, wherein after completion of a step of a surgical procedure corresponding to the trajectory, the processing circuitry is further configured to reactivate the cooperative mode for the robotic arm.

8. The system of any of claims 1-7, wherein the influence zone is a volume surrounding the key point corresponding to a step of a surgical procedure corresponding to the trajectory.

9. The system of any of claims 1-7, wherein the robotic arm is configured to operate below a maximum speed in each of the cooperative and autonomous modes, the maximum speed being higher in the cooperative mode than the autonomous mode.

10. A method for autonomous robotic control comprising:
generating a surgical plan including receiving a surgeon selection of a key point;
determining a trajectory, for a robotic arm, based on the key point, the trajectory including a position and orientation for the robotic arm;
identifying that the robotic arm has been moved, while in a cooperative mode, into an influence zone corresponding to the key point;
controlling the robotic arm to move, in an autonomous mode, to be aligned along the trajectory.

11. The method of claim 10, further comprising displaying the user interface on a display device, the user interface further including a visual representation of the trajectory.

12. The method of claim 10, further comprising receiving a surgeon selection to proceed with the autonomous movement along the trajectory before controlling the robotic arm to be aligned along the trajectory.

13. The method of claim 12, wherein receiving the surgeon selection includes receiving selection on the user interface of the key point from a plurality of planned key points corresponding to a plurality of planned trajectories.

14. The method of claim 10, further comprising receiving tracking information of movement of the robotic arm, and displaying, on the user interface, a position and orientation of the robotic arm corresponding to the tracking information.

15. The method of claim 10, further comprising receiving an intraoperative change to the trajectory before the autonomous movement, the change causing the robotic arm to autonomously move the robotic arm along the new trajectory.
